# EUROPEAN PATENT SPECIFICATION

(11) **EP 3 756 716 B1**
(45) Date of publication and mention of the grant of the patent: **22.03.2023**
(21) Application number: 20180569.4
(22) Date of filing: 17.06.2020
(51) Int. Cl.: A61M 16/06, A61M 16/20, A61M 16/00

(54) **MASK FOR RESPIRATORY THERAPY FOR PATIENTS**
ATEMSCHUTZMASKE FÜR PATIENTEN
MASQUE DE THÉRAPIE RESPIRATOIRE POUR LES PATIENTS

(30) Priority: 28.06.2019 IT 201900010425
(43) Date of publication of application: 30.12.2020
(73) Proprietor: Intersurgical S.P.A., 41037 Mirandola, Modena (IT)
(72) Inventor: ROSSI, Paolo, 41037 Mirandola (MO) (IT)
(74) Representative: Corradini, Corrado

(56) References cited:
- WO-A1-2007/045023
- WO-A1-2018/116040
- WO-A2-2006/113321
- US-A1- 2008 053 446
- US-A1- 2011 226 254
- US-A1- 2018 236 198

## Description

### TECHNICAL FIELD

The present invention relates to a mask for non-invasive respiratory therapy for patients, i.e. for the forced (mechanical) ventilation of patients, e.g. a so-called full-face mask. More in particular, the invention relates to a mask, e.g. a full-face mask, for performing CPAP or NIV therapies.

### PRIOR ART

For CPAP or NIV therapy, at home or at hospital, the use of masks that fall within three general categories is known.

A first category is that of nasal masks, i.e. that surround the patient's nose only, a second category is that of oronasal masks, i.e. that surround the patient's nose and mouth and a third category is the category of full-face masks, i.e. masks that surround the patient's whole face, i.e. their eyes, nose and mouth.

Full-face masks are generally comprised of a rigid, semi-rigid shell, e.g. obtained by moulding, or a floppy shell, which has an annular perimeter edge intended to come into contact, through the interposition of a seal, with the patient's face, i.e. with a perimeter thereof that inscribes the mouth and/or nose and possibly the eyes. An example of known full-face mask according the preamble of claim 1 is disclosed in document WO 2018/116040 A1.

A requirement of the prior art is that of improving the pneumatic seal between the shell, i.e. the perimeter edge thereof (and/or the seal) and the patient's face, especially when such masks - used for CPAP and NIV therapies - are subject to (high) internal pressure and must be worn for extended periods of time.

In fact, a loss of pressure inside the shell of the mask translates into a loss of effectiveness of the therapy (until the failure thereof) and a respiratory risk for the patient.

Within the context of such a need, a requirement is also perceived in masks of the known type to improve and guarantee the comfort for the patient, reducing as much as possible the risk of them developing ulcers or sores in the contact area between the perimeter edge of the shell (and/or the seal) and the patient's face.

An object of the present invention is to solve the requirements of the prior art, with a simple, rational and low-cost solution.

Such purposes are accomplished by the characteristics of the invention given in the independent claim. The dependent claims outline preferred and/or particularly advantageous aspects of the invention.

### DISCLOSURE OF THE INVENTION

The invention particularly makes available a mask for non-invasive respiratory therapy for patients, and is defined in independent claim 1.

Thanks to such solution, an effective and tight connection is enabled between the perimeter edge of the shell of the mask and the patient's face for the entire perimeter of the edge itself.

Furthermore, a reusable frame is made available - where possible - also when the shell is to be removed and replaced.

According to an aspect of the invention, the support frame can be configured to be fitted onto the shell and exert a pressure on the perimeter edge of the shell against the patient's face.

In particular, the support frame is configured to (directly or indirectly) contact o surround or be in proximity of and/or at the perimeter edge, preferably for the entire perimeter (o circumferential development) thereof, for instance in such a way to surround the entire perimeter edge.

Thanks to such solution, it is possible to make a quick and effective connection between the shell and the frame.

According to the invention, the support frame comprises at least one fixing point for the fixing of the support frame to a neck strap.

Thanks to such solution, the traction action exerted by the neck strap is discharged onto the annular frame, which uniformly distributes the pull thereof along its entire annular perimeter, allowing uniform traction to be exerted along the entire perimeter edge of the shell of the mask.

Again, the support frame may be rigid or semi-rigid, e.g. inextensible although flexible. Furthermore, according to one embodiment, the support frame can be made in a monolithic body.

Alternatively, the support frame can be made of numerous bodies joined together (e.g. consecutive along the perimeter or concentric).

Thanks to such a solution, it is possible to provide a support frame that can be easily adapted to various sizes or measurements of shells and/or that is easy to install, e.g. closable directly onto the shell positioned on the patient's face.

According to the invention the perimeter edge of the shell supports or comprises an annular seal. Advantageously, according to the invention the annular seal is adapted to be compressed, in use, between the patient's face and the support frame.

In particular, the support frame is configured to (directly or indirectly) contact o surround or be in proximity of and/or at the annular seal, preferably for the entire perimeter (o circumferential development) thereof, for instance in such a way to (directly) contact the entire circumferential development of the surface (namely, the surface opposed to the surface of the annular seal that is designed to contact the patient's face) of the annular seal.

In particular, the support frame is configured to compress, along the entire annular development, (the entire circumferential annular of) the annular seal onto the patient's face. Thanks to such solution, it is possible to maximize the hermetic sealing action exerted by the annular seal onto the face of the patient wearing the mask.

In a preferred embodiment, the annular seal may be of the inflatable type.

According to an aspect of the invention, the mask may comprise a neck strap associated with the support frame for the anchoring of the mask to the patient's head.

Again, according to the invention, the mask is of the full-face type, and the perimeter edge of the shell and the support frame being adapted to surround (within them) the patient's mouth, nose and eyes.

### BRIEF DESCRIPTION OF THE DRAWINGS

Further features and advantages of the invention will be more apparent after reading the following description provided by way of non-limiting example, with the aid of the accompanying drawings.

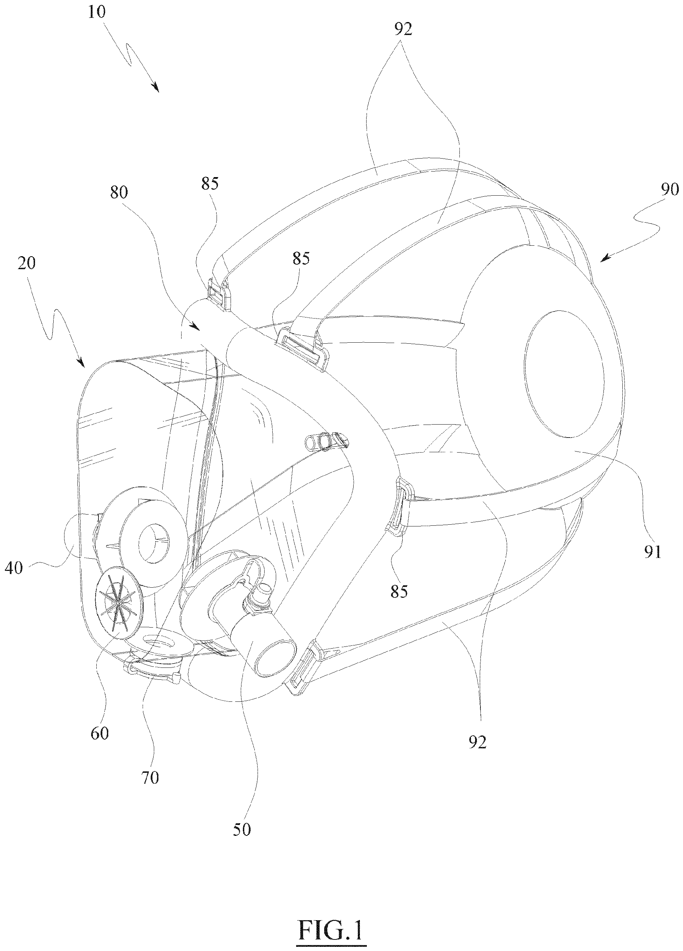

Figure 1 is an axonometric view of a first embodiment of a mask according to the invention.

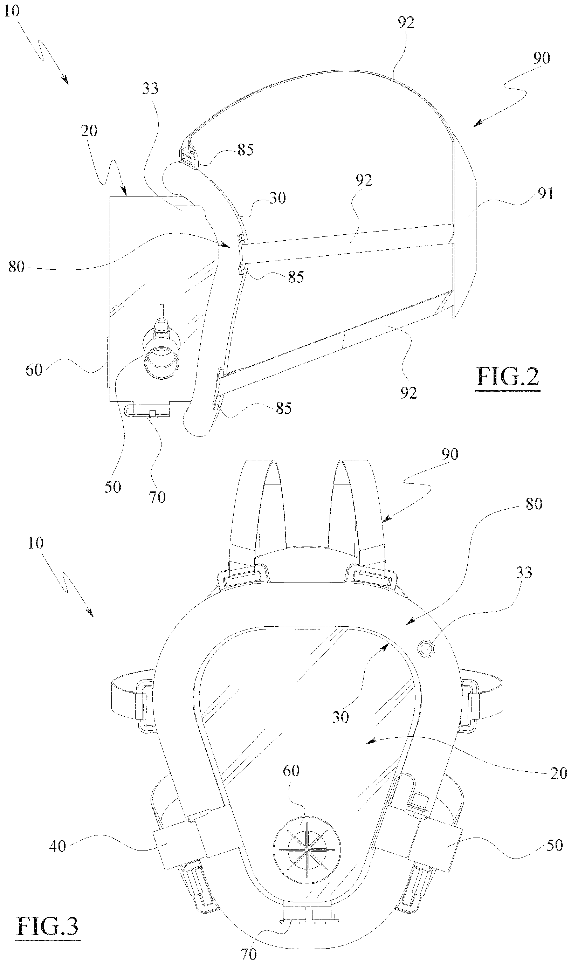

Figure 2 shows a side view of Figure 1.
Figure 3 shows a front view of Figure 1.

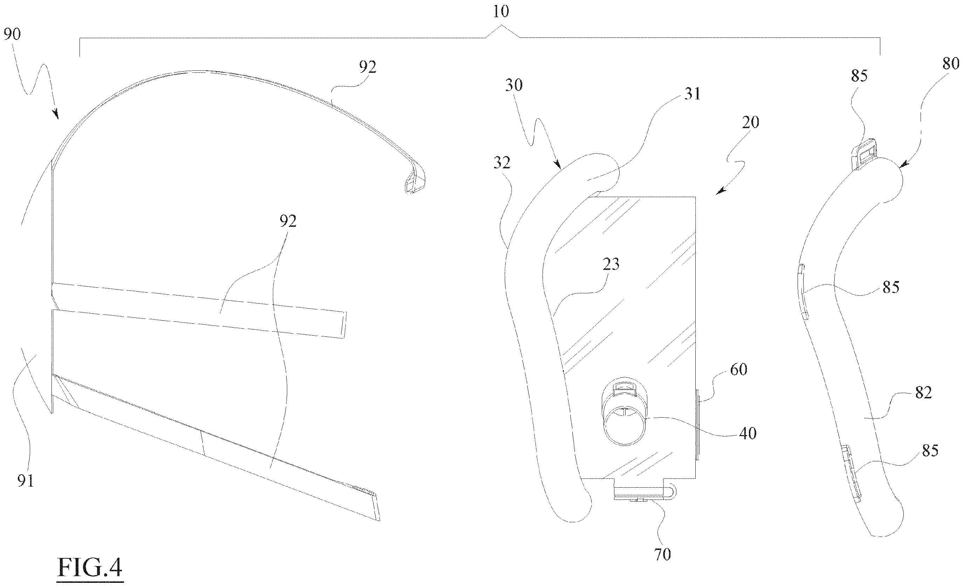

Figure 4 shows an exploded side view of Figure 1.

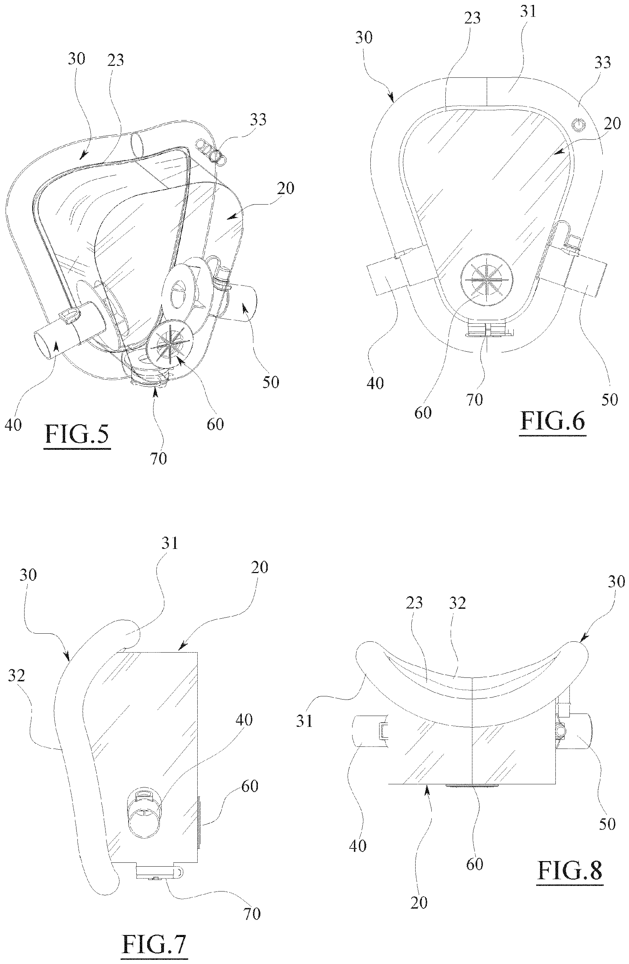

Figure 5 is an axonometric view of a shell of the mask of Figure 1.
Figure 6 shows a front view of Figure 5.
Figure 7 shows a side view of Figure 5.
Figure 8 is a plan view (from above) of Figure 5.

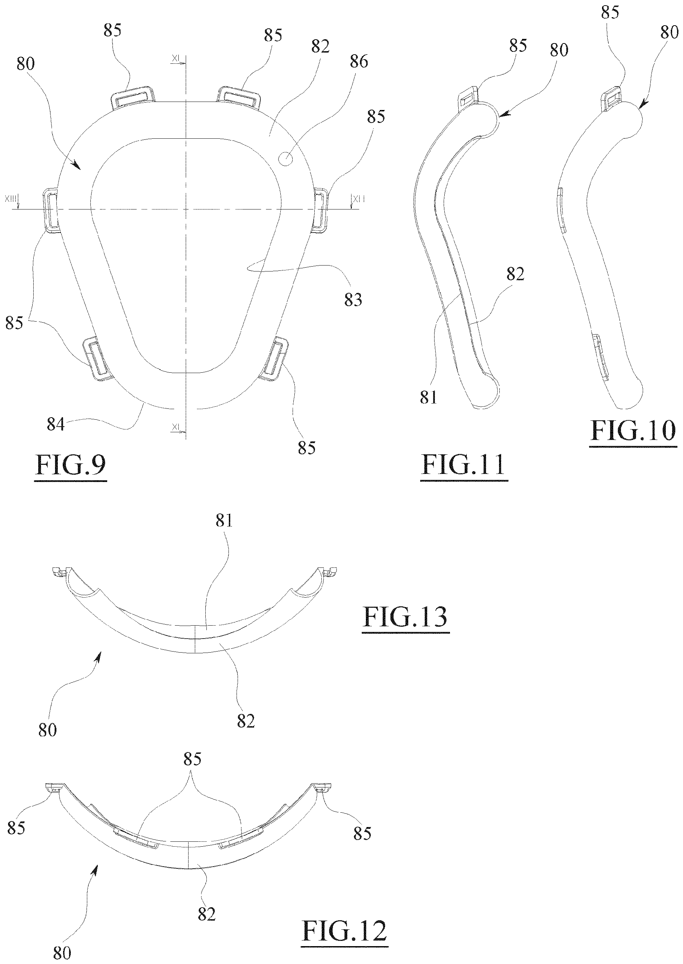

Figure 9 is a front view of a support frame of the mask of Figure 1.
Figure 10 shows a side view of Figure 9.
Figure 11 is a sectional view along the trace of section XI-XI of Figure 9.
Figure 12 is a plan view (from above) of Figure 9.
Figure 13 is a sectional view along the trace of section XIII-XIII of Figure 9.

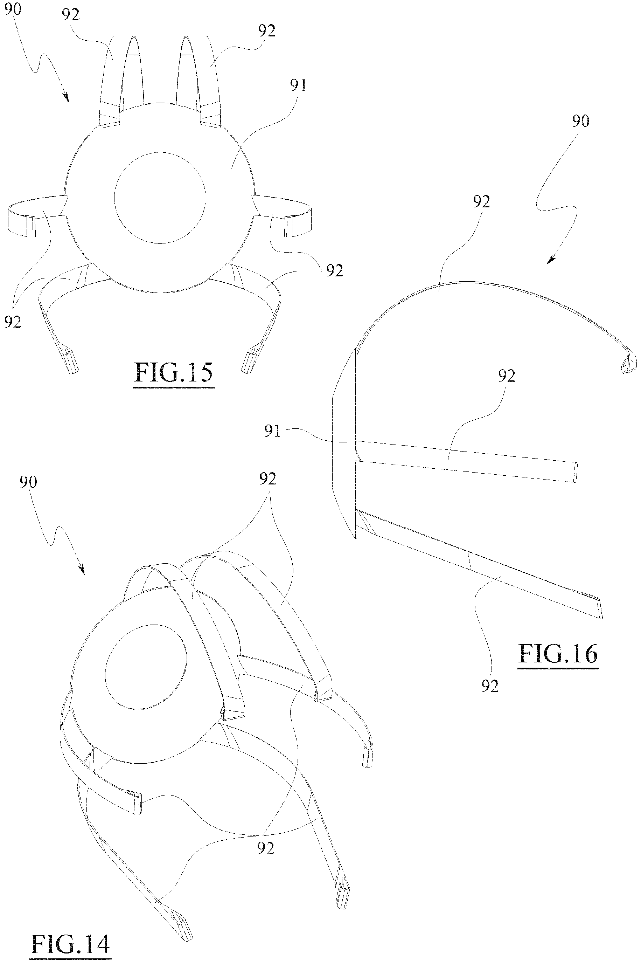

Figure 14 is an axonometric view of a neck strap of the mask of Figure 1.
Figure 15 shows a front view of Figure 14.
Figure 16 shows a side view of Figure 14.
Figure 17 is an axonometric view of a second embodiment of a mask according to the invention.
Figure 18 shows a side view of Figure 17.

### BEST WAY TO ACTUATE THE INVENTION

With particular reference to such figures, 10 indicates overall a mask, for example a full-face mask which means a mask adapted to surround (circumscribe and contain within it) the nose, mouth and eyes of a user, i.e. a patient.

It is not excluded that the mask 10 is of the nasal type, i.e. adapted to surround the patient's nose only, or oronasal, i.e. adapted to surround the patient's nose and mouth only. The mask 10 overall or in every part thereof (or in some of the parts of which it is composed) is for example disposable or for a single patient or, alternatively, it may be sterilized and/or placed in an autoclave and/or disinfected.

The mask 10 comprises a shell 20, which shell 20 is for example substantially concave, with concavity facing towards the patient's face.

The shell 20, therefore, has a concave inner side (or intrados) and an opposing convex outer side (or extrados).

The shell 20 is made of a gas- (and liquid-) impermeable material, i.e. that defines an impassable barrier for gases.

In a first embodiment shown in the figures, the shell 20 can be made overall of a flexible and inextensible material, e.g. with reduced (if not null) traction elasticity.

For example, the shell 20 is made of polyvinyl chloride (PVC) or another material.

In such embodiment the shell 20 of the mask 10 is for example generally made without a frame (frameless), i.e. without any rigid structural parts that determine overall a predefined undeformable or substantially undeformable shape thereof.

In practice, the shell 20, in such embodiment, can be bent or crumpled without this comprising the structural and functional integrity thereof.

The shell 20 overall is for example inflatable, inflatable meaning that it can pass alternatively between a deflated configuration and a (different) inflated configuration, wherein in the inflated configuration the volume of the shell 20 is different, preferably greater, than the volume of the same shell 20 in the deflated configuration.

For example, the shell 20 overall is adapted to pass from the deflated configuration to the inflated configuration due to the effect of the operating pressure (for CPAP or NIV therapy), which is greater than atmospheric pressure, at which a breathable gas is supplied to be made available for the inspiration of a user of the shell 20, as will appear more clearly below.

However, it is not excluded that a substantially rigid shaped or elastically deformable (shape memory) ring can be permanently associated with the shell 20, e.g. in an inner flap thereof.

It is not excluded that the shell 20 may be of the rigid or semi-rigid type, i.e. be shaped according to its own shape, e.g. unchangeable (without the loss of the structural integrity thereof or the common stresses to which it is subjected in operation such as, for example, the operating pressure at which a breathable gas is supplied to be made available for the inspiration of a user of the shell 20).

In such circumstances, the shell 20 may be made of a rigid or semi-rigid plastic material, e.g. polycarbonate (PC) or copolyester (PETG) or another suitable material.

In both embodiments, the shell 20 comprises a substantially closed loop shaped perimeter edge 23, e.g. shaped, which is adapted to come into contact (as will be better described below) with a front line of the patient's face that it surrounds and inscribes, e.g. the mouth, nose and eyes thereof.

The edge 23 has a substantially trapezoid shape (with rounded edges and/or shaped/convex edges), e.g. an isosceles trapezium, with a lower base placed at the bottom when operating (in the chin area) and a larger base placed at the top (in the forehead area).

In practice, a smaller lower base 23 is adapted to rest (although not directly) on the patient's chin (below their mouth), a larger upper base of the edge 23 is adapted to rest (although not directly) on the patient's forehead (above their eyes) and two sides that join the larger upper base to the smaller lower base are adapted to rest (although not directly) on the side of the face, i.e. around the eyes and cheeks (to the side of the eyes, nose and mouth).

The trapezoidal shape may also, for example, be substantially triangular or oval or circular according to requirements.

Generally, the edge 23 has a symmetrical shape with respect to a sagittal plane of the shell 20, which when the mask 10 is worn by the patient, actually coincides with the sagittal plane of the patient's face.

The shell 20, when the edge 23 rests on the patient's face, delimits a closed volume communicating, through the patient's mouth and nose, with the respiratory system (or with the lungs) of the actual patient.

The shell 20 has at least one at least partially optically transparent portion; in the example illustrated, the whole shell is at least partially optically transparent, preferably completely optically transparent.

The shell 20 overall or in every part thereof is for example disposable or for a single patient or, alternatively, it may be sterilized and/or placed in an autoclave and/or disinfected.

The mask 10 comprises an annular seal 30 fixed to the shell 20 and supported by it. The annular seal 30 is for example fixed (for example permanently or removably) to the shell 20 at or in proximity to the edge 23.

The annular seal 30 in practice surrounds the entire perimeter of the edge 23 of the shell 20.

In other words, the annular seal 30 in fact extends the edge 23 towards the patient's face when the mask 10 is worn and, therefore, defines the end portion thereof adapted to come into contact with the patient's face.

In practice, the edge 23 is adapted to come into contact with the patient's face through the interposition of the annular seal 30 (which remains interposed between them and compressed in operation).

The annular seal 30, for example when the shell 20 is rigid or semi-rigid, may be over-moulded or co-moulded with the edge 23 of the shell 20.

Alternatively, the annular seal 30 may be fixed, movably or removably, to the edge 23 of the shell 20.

For example, the annular seal 30 may be fixed to the edge 23 of the shell through a (snap) joint and/or, as illustrated in the first embodiment, through radio-frequency welding or heat-sealing.

The annular seal 30 is for example at least partially soft (yielding) and, for example, at least partially elastic/resilient.

The annular seal 30 comprises a ring-shaped body, e.g. with an overall homologous shape to the shape of the edge 23 of the shell 20, i.e. such as to surround the entire perimeter of the edge 23.

Preferably, but in a non-limiting way, the annular seal 30 projects radially outside of the (perimeter defined by the) edge 23, so as to extend it in the radial direction (for the entire perimeter).

The annular seal 30 is preferably an inflatable seal, e.g. like an inflatable cushion, before the use of the mask 10.

The annular seal 30 has an internal chamber, e.g. an inflatable air chamber.

The annular seal 30 is therefore adapted to pass alternatively between a deflated configuration (and for example floppy/flexible and with a reduced axial dimension) and a (different) inflated configuration (and for example more rigid and with a larger axial dimension), in which in the inflated configuration the volume of the annular seal 30 is different, preferably larger, than the volume of the annular seal 30 in the deflated configuration.

The annular seal 30, when in the deflated configuration, can be bent and/or crumpled, whereas when it is in the inflated configuration it has a certain (non null) resistance to bending, i.e. it tends to maintain its annular shape when subjected to compression and/or bending and/or torsion stress.

The annular seal 30, for example, is defined by a first annular layer 31 for example flat (substantially trapezoid shaped, as defined above for the edge 23) and by a second annular layer 32 for example flat (substantially trapezoid shaped homologous to the first annular layer 31) joined together (sealingly), for example exclusively, at an outer perimeter portion and/or an inner perimeter portion.

For example, the first annular layer 31 and the second annular layer 32, at the aforesaid perimeter portions, are joined together permanently by radio-frequency welding or heat sealing.

The inner chamber is defined by the volume contained between the first annular layer 31 and the second annular layer 32 joined at the outer perimeter portion and the inner perimeter portion of the annular layers 31 and 32 themselves.

The first annular layer 31 is for example axially proximal to the shell 20 and the second annular layer 32 is axially distal from the shell 20.

Each annular layer 31,32 has an inner face, facing towards the inside of the inner chamber and an opposing outer face.

The outer face of the second annular layer 32 is the portion of the annular seal 30 that defines a resting and (forced) contact surface with the face of the patient and user of the mask 10 when it is worn.

The annular seal 30, for example at least the outer face of the second annular layer 32 (or both the annular layers) is made of an elastically yielding and/or soft plastic material, e.g. polyurethane (PU) or polyvinyl chloride (PVC), e.g. with high compatibility with the patient's epidermis.

The annular seal 30 is fixed (through radio-frequency welding or heat sealing) to the edge 23 of the shell 20.

The annular seal 30, for example, comprises an inflation valve 33 through which the inner chamber can be connected to the outside for the inflation and/or deflation thereof.

The inflation valve 33 is for example fixed to the annular seal 30 on the opposite side with respect to the resting surface intended to rest on the patient's face.

For example, the inflation valve 33 is placed outside the volume contained by the shell 20 (and by the patient's face when the mask 10 is worn by the actual patient).

For example, the inflation valve 33 is fixed to the (outer face of the) first annular layer 31, e.g. in a portion thereof defined externally (in the radial direction) with respect to the edge 23.

The inflation valve 33 comprises a tube sealingly fixed (for example through radio-frequency welding or heat sealing) to the perimeter edge of a through hole made in one of the annular layers 31,32 and shutter means, e.g. a plug or a gripper (if the tube is deformable).

For example, the inflation valve 33 may be a Schrader type valve or a membrane valve or an automatic one-way valve that prevents the spontaneous exit of air from the internal chamber or the like.

The annular seal 30, when in the inflated configuration, has a certain softness (or axial compressibility) and adaptability to the shape of the patient's face, i.e. it has means for compensating for the clearance between the edge 23 of the shell 20 and the patient's face.

A lip, for example a flexible lip, can be associated with the annular seal 30.

However, it is not excluded that the annular seal 30 is not of the variable volume or configuration type, i.e. that it is not inflatable but is a fixed configuration seal, e.g. only elastically and/or plastically deformable.

In that case, the annular seal 30 may be made of soft, spongy, viscoelastic or viscoplastic material.

Finally, it is not excluded that the annular seal 30 may be made of a single layer made of an elastically yielding and/or soft plastic material, e.g. polyurethane (PU) or polyvinyl chloride (PVC), e.g. with high compatibility with the patient's epidermis.

The annular seal 30 overall or in every part thereof is for example disposable or for a single patient or, alternatively, it may be sterilized and/or placed in an autoclave and/or disinfected.

The mask 10 then comprises intake means of a breathable gas, e.g. air or a mixture of air and oxygen or oxygen, into the volume contained by the shell 20 and the annular seal 30 (and the patient's face when the latter is wearing the mask 10).

In the example shown, the intake means comprise an inlet conduit 40 for the intake of air. The inlet conduit 40 (which for example has a substantially rectilinear or curvilinear longitudinal axis and a substantially circular cross section) is advantageously fixed, at a constrained end thereof, non-removably to the mask 10, in particular to the shell 20 thereof. For example, the inlet conduit 40 is firmly fixed (sealingly) to the entire perimeter edge of a through hole made in the shell 20, and for example exits in a centrifugal/radial direction therefrom, washing carbon dioxide uniformly away from the patient's mouth and nose. The inlet conduit 40 may for example be provided, at the end that leads into the volume contained by the shell 20 and by the annular seal 30 (and the patient's face), with a diffuser.

Furthermore, the inlet conduit 40 may be provided with a pressure tap connector, e.g. tapered in a substantially radial direction with respect to the inlet conduit itself, with which a pressure gauge can be associated or is closed by a relevant plug.

The free end (distal from and external to the shell 20) of the inlet conduit 40 has, for example, standard dimensions (e.g. 22M) for connection to usual instruments for supplying breathable gas such as air, air and oxygen or oxygen, such as cylinders, conduits, flow meters, Venturi meters or similar devices.

The inlet conduit 40 and the shell 20 actually constitute an inseparable single body and, for example, are fixed firmly through welding or through gluing or another non-soluble fixing technique or are made in a single body.

The inlet conduit 40 is, for example, fixed to the shell 20, for example in any part thereof, preferably at one or two of the lateral sides of the shell 20 (frontally superposed with a cheek - e.g. right - of the patient when the latter is wearing the mask 10).

For example, the inlet conduit 40 is arranged so that its constrained end (that leads into the internal volume contained by the shell 20) is in proximity to the patient's oronasal area, preferably but not necessarily to the side of it.

The mask 10 then comprises outflow means of an expired gas, e.g. carbon dioxide, for the exit from the volume contained by the shell 20 and by the annular seal 30 (and the patient's face when the latter is wearing the mask 10) of such expired gas.

In the example shown, the outflow means comprise an outlet conduit 50 for the exit of the expired gas.

The outlet conduit 50 (which for example has a substantially rectilinear or curvilinear longitudinal axis and a substantially circular cross section) is advantageously fixed, at a constrained end thereof, irremovably to the mask 10, i.e. to the shell 20 thereof (e.g. in a different and distanced point with respect to where the inlet conduit 40 is fixed).

For example, the outlet conduit 50 is firmly fixed (sealingly) to the entire perimeter edge of a through hole made in the shell 20, and for example exits in a centrifugal/radial direction therefrom.

The outlet conduit 50 may for example be provided, at the end that leads into the volume contained by the shell 20 and by the annular seal 30 (and the patient's face), with a diffuser.

Furthermore, the outlet conduit 50 may be provided with a connector e.g. tapered in a substantially radial direction with respect to the outlet conduit itself, with which a relevant instrument can be associated or is closed by a relevant plug.

The free end (distal from and external to the shell 20) of the outlet conduit 50 has, for example, standard dimensions (e.g. 22F or 22M) for connection to usual instruments for the exit of the expired gas, including for example an exit tube, e.g. connected to a ventilator (for NIV therapy), or a PEEP valve (for CPAP therapy) and/or other similar devices. The outlet conduit 50 and the shell 20 actually constitute an inseparable single body and, for example, are fixed firmly through welding or through gluing or another non-soluble fixing technique or are made in a single body.

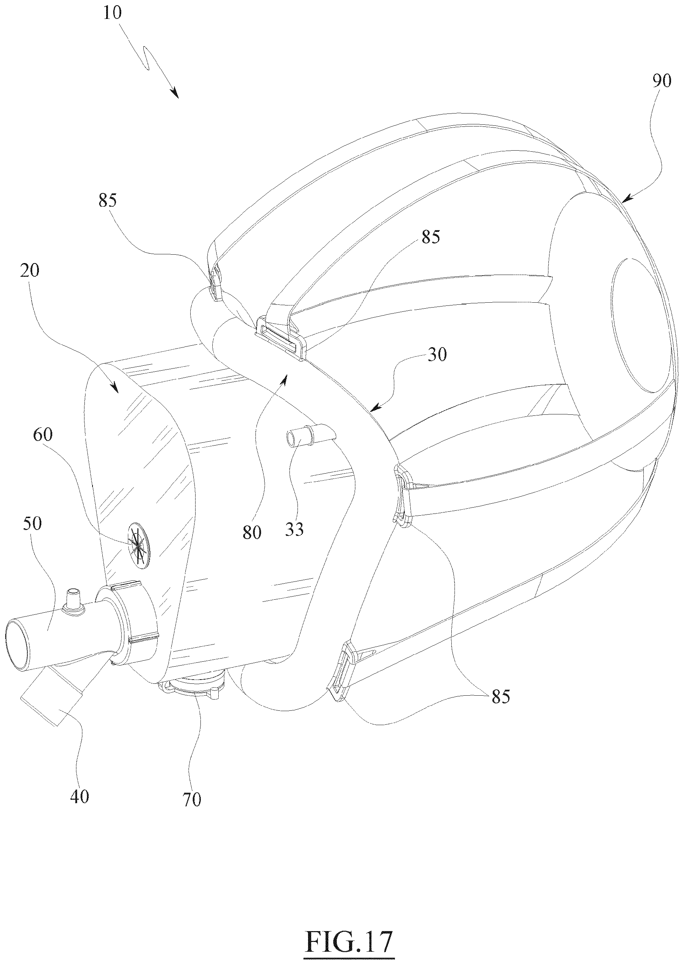

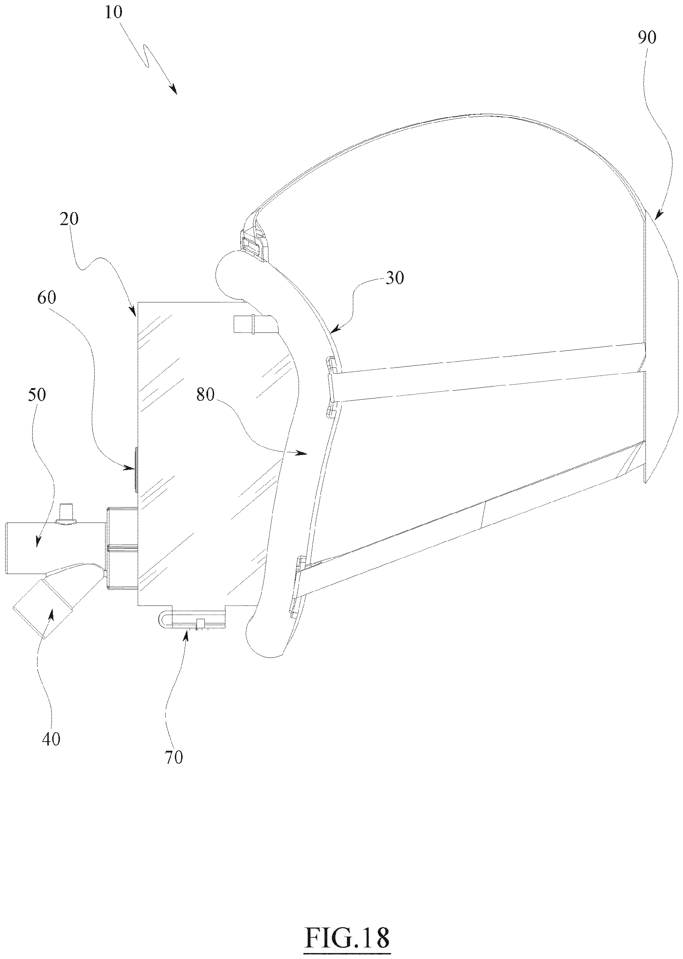

In the example illustrated the outlet conduit 50 is fixed to the shell 20, for example in any part thereof, e.g. at one of the two lateral sides of the shell 20 (frontally superposed with a cheek - e.g. left - of the patient when the latter is wearing the mask 10), e.g. the lateral side opposite the one in which the inlet conduit 40 is placed (e.g. substantially symmetrical with respect to it in relation to a sagittal plane of the shell 20 and of the patient). For example, the outlet conduit 50 is arranged so that its constrained end is in proximity to the patient's oronasal area, preferably but not necessarily to the side of it. Furthermore, it is not excluded that the inlet conduit 40 and the outlet conduit 50, as illustrated in Figures 17-18, are integrated into a single Y-shaped connector, in which a first free end (distal from the shell 20) defines the inlet conduit 40, a second free end (distal from the shell 20) defines the outlet conduit 50 and a third constrained end (proximal to the shell itself) is connected to a (single) opening (e.g. provided with a cylindrical connector, preferably axially rotatable) provided on the shell 20 (e.g. in a front area thereof in front of the patient's mouth when the mask 10 is worn by the actual patient). The mask 10 can further comprise an anti-suffocation valve 60, i.e. a two-directional valve that can place in communication the volume contained by the shell 20 and by the annular seal 30 (and the patient's face) with the volume external thereto, in the event of emergency, i.e. when the pressure of the breathable gas inside such volume drops below a minimum threshold value, e.g. greater than (or equal to) atmospheric pressure and less than the useful operating pressure for the patient's NIV or CPAP therapy, which will be better described below, to which the breathable gas is supplied through the intake means. The anti-suffocation valve 60 can be non-removably (or removably) associated with the shell 20, e.g. with any portion of the shell 20 (better if in proximity to the patient's mouth). The anti-suffocation valve 60 is absolutely identical to the one illustrated and described in international patent application No. WO 2018/116040 in the name of the same Applicant.

For example, the anti-suffocation valve 60 comprises a valve body which is fixed, for example, non-removably, to the entire perimeter of a through hole made in the shell 20. The valve body is substantially cylindrical (with reduced height), hollow and has a bottom wall external to the shell 20, for example provided with an attachment flange fixed to the perimeter of the through hole, and an opposing bottom wall internal to the shell 20 (to the internal volume thereof, i.e. to its concavity).

The internal bottom wall is, for example, substantially planar and has a through opening, e.g. a plurality of through openings defining a passage opening for air.

The anti-suffocation valve 60 also comprises a through opening placed on the outer bottom wall.

The outer bottom wall is, for example, substantially planar and, in the example, the through opening on the outer bottom wall (like the one made in the inner bottom wall) is made overall of a plurality of holes, e.g. with a circular sector and concentric conformation, which are divided from each other by radial ribs.

Advantageously, the total area of the through opening (on the outer bottom wall and/or on the inner bottom wall) is substantially (for example from 5 to 20 times) larger than the passage section of a 22M or 22F standard connection connector.

The radial ribs are joined at their central end and are joined to a peripheral edge of the outer bottom wall at the peripheral end thereof.

For example, the ribs are centred on the axis of the valve body.

The outer bottom wall, at the central joining area of the ribs, comprises a central through hole, e.g. substantially circular.

The anti-suffocation valve 60 further comprises a shutter positioned inside the valve body and movable from an opening position of the through opening on the outer bottom wall to a closing portion thereof, in contrast to thrust means.

In particular, the shutter comprises a disc-shaped body, which is arranged substantially parallel to the outer (and inner) bottom wall.

The disc-shaped body has a larger diameter than the diameter of the through opening, so as to be able to completely close it.

The disc-shaped body also has a smaller diameter than the inner diameter of the valve body, so that a radial gap is defined between the periphery of the disc-shaped body and the inner side wall of the valve-shaped body.

In practice, in the closing position at least the outer perimeter edge of the disc-shaped body is adapted to come into forced contact, thanks to the action of the positive pressure inside the shell 20 (i.e. the volume contained between the shell 20, the annular seal 30 and the patient's face), with at least the inner peripheral edge of the outer bottom wall, closing (from the inside of the valve body) substantially sealingly, the through opening on the outer bottom wall, in contrast to the thrust means.

The disc-shaped body is for example made of at least a substantially flexible material, such as a plastic material, or alternatively could be rigid and have a perimeter edge projecting towards the outer bottom wall made of substantially yielding material, such as rubber or plastic for creating the seal.

The shutter comprises a central stem projecting from the centre of the disc-shaped body, which comprises an end fixed to the disc-shaped body itself; the opposite free end is for example provided with an enlarged portion, e.g. a knob.

The central section of the central stem is for example substantially cylindrical and is adapted to be threaded substantially to measure into the central hole of the outer bottom wall, so as to slide along it between two stroke end positions.

The first stroke end (closing) position is defined by the contact inside the valve body of the disc-shaped body with the outer bottom wall and defines the closing position of the shutter; the second stroke end position is defined by the contact on the outside of the valve body, the enlarged portion of the central stem with the outer bottom wall (the joining area of the ribs) and defines the opening position of the shutter.

In practice, the shutter is slidably associated, along the axial direction of the valve body, with the inside of the valve body, between a position close to the bottom wall (closing position) and a position distanced therefrom (opening position).

The thrust means are configured to generate a thrust force on the shutter to bring it towards the opening position.

The thrust means, in the example illustrated, comprise an elastic element and preferably a spring, e.g. a compression spring, which is interposed between the disc-shaped body of the shutter and the outer bottom wall (in the example the joining area of the ribs that surrounds the central hold) of the valve body.

In practice, the spring is a helical spring that is threaded onto the central section of the central stem inside the valve body.

However, it is not excluded that the spring may be a torsion spring, a disc spring or another type of spring or that the thrust means may be of a different kind, e.g. of the magnetic type.

Optionally, the valve body may comprise, instead of the cylindrical element and the inner bottom wall, a cross element that performs the same function, i.e. it is adapted to keep the shutter in position inside the valve body defining a sliding seat thereof contained radially and axially and to let the air that passes through the through opening made in the outer bottom wall flow out.

Alternatively, the anti-suffocation valve 60 may be removably associated, according to requirements, for example with a completely analogous (and distinct) auxiliary conduit to the inlet conduit 40 or to the outlet conduit 50 that may be provided in the shell 20 of the mask 10.

The mask 10 can further comprise a further through hole made in the shell 20 to whose perimeter edge a connection element 70 is fixed, e.g. of the cable gland type (such as SNG or the like) or another connector for various instruments adapted for the patient's therapy, which connect the volume external to the mask 10 with the volume contained by the shell 20 and by the annular seal 30 (and by the face of the patient wearing the mask 10) and that can be closed by a membrane and/or a cover according to requirements. The connection element 70 and the shell 20 actually constitute an inseparable single body and, for example, are fixed firmly through welding or through gluing or another non-soluble fixing technique, e.g. made by co-moulding or over-moulding or made in a single body. In this way, the connection element 70 is in fact integrated into the shell 20 of the mask 10, i.e. to one of the portions of which it is made.

In the example illustrated the connection element 70 is fixed to the shell 20, e.g. in any part thereof, preferably but in a non-limiting way at a smaller (lower) base thereof placed in proximity to the patient's chin/mouth.

The shell 20 may have one or more openings adapted for the connection of one or more respiratory circuits, one- or two-directional anti-asphyxia safety valves, overpressure safety valves, PEEP valves, passage ports for probes, catheters, external or integrated sensors for monitoring parameters; furthermore, the shell 20 may have integrated components for monitoring the pressure and concentration of the breathable gases, may have integrated elements for the removal of the gases or the increased circulation of the gases inside it, may have integrated elements for generating pressure inside it (diaphragms, intentional leaks, mechanical PEEP valves or expiratory resistances).

The shell 20 overall or in every part thereof is for example disposable or for a single patient or, alternatively, it may be sterilized and/or placed in an autoclave and/or disinfected.

The mask 10 further comprises a support frame 80, which for example has a (closed) annular shape overall.

The support frame 80 is removably associated with the shell 20 (and/or the annular seal 30), as will appear more clearly below.

The support frame 80, in particular, is configured to exert pressure on the perimeter edge 23 of the shell 20 (and preferably on the annular seal 30) against the patient's face, as will be better explained below.

For example, the support frame 80 has an inner face 81, i.e. facing towards the patient's face when worn by the actual patient, and an opposing outer face 82.

The support frame 80 surrounds and defines a through opening 83, appropriately shaped, which is adapted to be threaded (axially and/or crossed) or engaged by at least one portion of the shell 20.

In particular, the support frame 80 has an outer edge 84 (shaped), which defines the outer perimeter of the support frame itself, and an inner edge (shaped), which delimits and defines the through opening 83.

The outer edge 84 for example does not surpass (unless in a limited way) the outer peripheral edge of the annular seal 30 and the inner edge (which delimits the through opening 83) is substantially analogous or slightly larger than the edge 23, i.e. than the inner peripheral edge of the annular seal 30.

For example, the support frame 80 is substantially plate-shaped or has a reduced thickness, thickness meaning the distance between the inner face 81 and the outer face 82.

The through opening 83 has a shape such as to surround the edge 23 of the shell 20, e.g. it has a homologous shape (slightly larger) to the shape of the edge 23 of the shell 20, so as to be engaged to measure therewith, when the support frame 80 is connected to the shell 20.

For example, the through opening 83 may be of a variable size, preferably as a function of the size of the shell 20 and/or of the annular seal 30, e.g. as a function of the size and/or shape of the edge 23 of the shell 20 and/or of the annular seal 30.

In a preferred embodiment shown in the figures, the inner face 81 of the support frame 80 is concave (overall).

In the example, the opposing outer face 82 is convex, but it is not excluded that it may be flat or even convex too (or variously configured according to requirements).

In particular, the inner face 81 is configured to define an abutment (and/or resting) surface for at least one portion of the annular seal 30, i.e. for the portion thereof projecting radially towards the outside of the edge 23 of the shell 20, in more detail for the first annular layer 31 (when the annular seal 30 is inflated).

In other words, the inner face 81 defines a crib in which the (portion projecting radially towards the outside of the edge 23 of the shell 20 of the) annular seal 30 is at least partially housed (removably).

Preferably, the support frame 80 comprises a housing seat 86, e.g. defined by a through hole, in which the inflation valve 33 is (at least partially) housed.

In the example, the inflation valve 33 is threaded axially into the housing seat 86, passing from one side to the other of the support frame 80.

The support frame 80 is preferably substantially rigid overall, i.e. undeformable by the stress to which it is normally subjected (in use).

However, it is not excluded that the support frame 80 may be semi-rigid overall, i.e. have a predetermined (elastic and/or plastic) deformability to bending and/or torsion (although it is for example substantially inextensible if subjected to traction and/or compression stress).

In that case, the support frame 80 is given a certain amount of adaptability to the shape of the patient's face and/or the annular seal 30 and/or the edge 23 of the shell 20 to which it is coupled.

The support frame 80 in the example illustrated is made in a single body (i.e. a single part), e.g. obtained by the injection moulding of a synthetic material (e.g. a polymer).

However, it is not excluded that the support frame 80 may be made of various separate bodies from each other, e.g. permanently or temporarily (demountable and remountable). For example, it is possible to envisage that the support frame 80 may be made by joining various sectors (joined together at their heads to define an overall annular "frame-like" body).

For example, the various sectors could be fixed to each other (for example rigidly) through joints and/or constraints (including for example at least one hinge or another constraining system).

Alternatively or additionally, it is possible to envisage the support frame 80 being made through joining various annular sub-frames, e.g. concentric, joined together perimetrally to define overall and individually an annular "frame-like" body).

For example, the various sub-frames could be fixed to each other (e.g. rigidly) through (perimeter) joints and/or constraints.

In that case, it is possible (simply and quickly) to make the through opening 83 have a variable size, as the inner edge of each sub-frame can define a respective through opening 83 with a different size from the others.

At least one fixing point 85 is provided on the support frame 80, preferably a plurality of fixing points 85 distributed along the (outer) perimeter of the support frame 80.

Each fixing point 85 comprises an end constrained (e.g. movably or removably) to the support frame 80, e.g. at or in proximity to the outer edge 84 thereof, and an opposing free end, e.g. facing or projecting (in the radial direction) from (the outer edge 84 of) the support frame 80.

For example, each fixing point 85 is fixed rigidly to the support frame 80 and, for example, is made in a single body therewith.

However, it is not excluded that each fixing point 85 or one or more of them can be adjustably fixed in position to the support frame 80, e.g. slidably, along the outer edge 84 thereof.

Preferably, but in a non-limiting way, each fixing point 85 comprises or is composed of a (through) eyelet.

It is not excluded that each fixing point 85 may comprise or be composed of a slot or a hole or a hook or, even, a layer of velcro (female or male) or a button or other quick coupling system.

In the example, the support frame 80 comprises a plurality of fixing points 85, e.g. separate from one another and placed in predetermined positions along the perimeter of the outer edge 84 of the support frame 80 itself.

For example, the support frame 80 comprises two right fixing points 85, e.g. (vertically) separate from each other. For example, one of the two right fixing points 85 is placed at the top, i.e. it is placed (or branches off, for example, towards the outside in a substantially centrifugal direction) for example in proximity to or at a vertex to the largest base of the support frame 80 (i.e. of the outer edge 84), and a right fixing point 85 is placed at the bottom, i.e. it is placed (or branches off, for example, towards the outside in a substantially centrifugal direction) for example in proximity to or at a vertex to the smallest base of the support frame 80 (i.e. of the outer edge 84).

Then, in the example the support frame 80 also comprises two left fixing points 85, symmetrical to the right fixing points with respect to a sagittal plane of the support frame 80, i.e. of the mask 10 (in fact coinciding with the sagittal plane of the patient wearing the mask 10).

Furthermore, the support frame 80 can comprise an upper fixing point 85 (or various upper fixing points 85), each of which is for example placed (or branches off for example, towards the outside in a substantially centrifugal direction) for example in proximity to or at (an intermediate point) of the largest base of the support frame 80 (i.e. of the outer edge 84).

For example, when there is only one upper fixing point 85 it is placed at a mid point of the largest base of the support frame 80 (i.e. of the outer edge 84), i.e. it lies and is cut (in half) by the sagittal plane of the support frame 80, i.e. of the mask 10.

In the example illustrated, the support frame 80 comprises two upper fixing points 85, which are separated from each other along the flanking direction along the upper outer edge 84 of the support frame 80 and, preferably, they are symmetrical with respect to the sagittal plane of the support frame 80, i.e. of the mask 10 (in fact coinciding with the sagittal plane of the patient wearing the mask 10).

In practice, the support frame 80 can have a number of fixing points equal to 3, 4 or 5 or 6.

It is not excluded that there may be any number of fixing points according to requirements. Furthermore, it is not excluded that the only continuous fixing point may be provided along the entire outer edge 84 of the support frame 80 or for a greater percentage than 50% thereof.

The support frame 80 overall or in every part thereof is for example disposable or for a single patient or, alternatively, it may be sterilized and/or placed in an autoclave and/or disinfected.

The mask 10 further comprises a neck strap 90, which is adapted to be wrapped around the back and/or the top of the patient's head (in the area of the nape of the neck) for retaining the shell 20 (and the annular seal 23) at the face of the actual patient (in close contact therewith), as will be better described below.

In more detail, the neck strap 90 is configured to be anchored to the support frame 80 for exerting traction thereon (and therefore through it on the shell 20 and on the annular seal 23) to press the shell 20, i.e. the annular seal 30) onto the face of the patient wearing the mask 10.

The neck strap 90 is for example adapted to be fixed, for example removably or permanently, to each fixing point 85 of the support frame 80.

The neck strap 90 comprises a central band 91 e.g. circular or rectangular shaped (or of any other shape), e.g. made of fabric or non-woven fabric (having a soft side, e.g. velvet ribbon, intended to come into contact with the nape of the patient's neck).

The neck strap 90 comprises a plurality of arms 92, e.g. each branching off (radially) from the central band 91, each of which is adapted to be constrained (permanently or removably) to a respective fixing point 85 of the support frame 80.

Each arm 92 has an end constrained to the central band 91, e.g. at or in proximity to an outer perimeter edge thereof, and an opposing free end.

Each arm 92 can be adjusted in length, e.g. at a constrained end or a free end thereof (or an intermediate section between the constrained end and the free end).

In fact, the neck strap 90 is adapted to be wrapped around the back of the patient's head (the nape of the neck) so as to retain and anchor the mask 10 (i.e. the shell 20 by means of the support frame 80 associated therewith) firmly to the patient's face, so that the annular seal 30 is pulled in forced contact towards the patient's head so as to exert a hermetic seal for the gases (breathable and breathed) present in the internal volume of the mask 10 contained by the shell 20, the annular seal 30 and the face of the actual patient. For example, the neck strap 90 has at least one left arm 92 (in the example two) and at least one right arm 92 (in the example two) which is symmetrical to the respective left arm 92 with respect to a sagittal plane of the neck strap 20 (substantially coinciding with the sagittal plane of the mask 10 and of the patient when being worn).

For example, each left or right arm 92 has a longitudinal extension (from the constrained end to the free end) which moves away from the sagittal plane of the neck strap itself.

The neck strap 90 may also comprise an upper arm 92 or a plurality of upper arms 92 (e.g. 2), each of which is for example placed (or branches off, for example, towards the outside in a substantially centrifugal direction) for example in proximity to or at an intermediate point between the right and left arms 92 of the central band 91.

In practice, the upper arm 92, when single, has a longitudinal axis parallel to and lying on the sagittal plane of the mask 10 and/or of the patient wearing it.

In the example illustrated, the neck strap 90 comprises two upper arms 92, which are separated from each other along the flanking direction along the outer periphery of the central band of the neck strap 90 and, preferably, they are symmetrical with respect to the sagittal plane of the neck strap 90, i.e. of the mask 10 (in fact coinciding with the sagittal plane of the patient wearing the mask 10).

Each left arm 92 is adapted to be fixed removably or permanently, to a respective left fixing point 85 of the support frame 80.

Each right arm 92 is adapted to be fixed removably or permanently, to a respective right fixing point 85 of the support frame 80.

Each upper arm 92 can be fixed removably or permanently, to a respective right fixing point 85 of the support frame 80.

It is not excluded that the neck strap 90 may be of the bonnet type, a front edge of which is connected (continuously) to the outer edge of the support frame 80 (e.g. to a greater percentage than 50% thereof).

Each arm 92 comprises, for example, a fixing element, e.g. at a free end thereof.

A fixing element may comprise a first layer of velcro (e.g. male) fixed (e.g. by sewing) to a side of the arm 92 and adapted to be coupled to a second layer of velcro (female) fixed (e.g. by sewing) to a (same) side of the arm 92 so as to define a ring (e.g. variable width and length).

In practice, the fixing element is adapted to be coupled or constrained to the respective fixing point 85 of the support frame 80, e.g. entering into the eyelet thereof and closing in a ring around it.

Alternatively or additionally, each fixing element can comprise a button or a hook or a layer of velcro (male) or another quick coupling element (such as areas made of fabric, silicone or rubber or another semi-rigid plastic material), adapted to be coupled to the (respective) fixing point 85 of the support frame 80.

For example, the length of the neck strap 90 (i.e. of the arms 92 and/or of the central band 91) can be adjustable.

The neck strap 90 overall or in every part thereof is for example disposable or for a single patient or, alternatively, it may be sterilized and/or placed in an autoclave and/or disinfected.

In light of the above, the operation of the mask 10 is as follows.

The mask 10, for example, is initially in a deflated condition thereof, in which for example the annular seal 30 (if inflatable) and the shell 20 (if floppy/deformable) are deflated. First of all, the annular seal 30 can be inflated to a desired inflation pressure.

In this way, the annular seal 30 gives a first pre-shape (substantially trapezoidal) to the shell 20 (i.e. to the edge 23 thereof), such as to be able to inscribe an area of the patient's face, e.g. In the case of a full-face mask 10 containing their eyes, nose and mouth.

At this point, the support frame 80 is fitted onto the shell 20, i.e. by threading the shell 20 into the through opening 83 until the entire circumferential development of (the entire outer face of) the annular seal 30 abuts (in contact) against the support frame 80 itself. In other words, the (entire) inner face 81 of the support frame 80 is brought into contact with the annular seal 30, i.e. with the (entire outer face of the) first annular layer 31. With the support frame 80 thus anchored to the shell 20, it is possible to connect the inlet conduit 40 and the outlet conduit 50 to the respective breathable gas intake and breathed gas evacuation circuits and join the neck strap 90 to the support frame 80, e.g. by joining one or more of the arms 92 of the neck strap 90 to one or more of the respective fixing points 85 of the support frame 80.

At this point it is possible to put the mask 10 onto the patient's face making sure that the annular seal 30 adheres, in forced contact, to the patient's face due to the pull exerted by the neck strap 90 onto the nape of the patient's neck and the compression exerted by the support frame 80 onto the edge 23, i.e. onto the annular seal 30 (towards the patient's face).

In other words, the annular seal 30 is compressed (for the entire circumferential development thereof) by (the force exerted by) the neck strap 90 between the support frame 80 and the face of the patient wearing the mask 10, increasing the seal of the annular seal 30 within the entire perimeter thereof.

The adduction of breathable gas at a therapeutic pressure greater than atmospheric pressure, e.g. comprised between 2.5 and 20 cm/H₂O, allows the shell 20 (when of the floppy/deformable type) to inflate into its inflated configuration and, then, complete the passage into the inflated configuration of the mask 10 overall.

Furthermore, the adduction of breathable gas at a therapeutic pressure greater than atmospheric pressure allows the administration to the patient of an effective CPAP or NIV therapy.

The support frame 80 allows gas leaks at the interface between the annular seal 30 and the patient's face to be reduced or prevented.

The invention thus conceived is susceptible to several modifications and variations, all falling within the scope of the inventive concept.

Moreover, all the details can be replaced by other technically equivalent elements.

In practice, the materials used, as well as the contingent shapes and sizes, can be whatever according to the requirements without for this reason departing from the scope of protection of the following claims.

## Claims

1. A full-face mask (10) for non-invasive respiratory therapy for patients, which comprises:
- a shell (20) provided with a closed loop perimeter edge (23) supporting an annular seal (30) adapted to come into forced and fluid-tight contact with an area of the patient's face and surround the patient's mouth, nose and eyes;
- intake means (40) of a breathable gas placed on the shell (20) for the intake of breathable gas into a volume contained between the shell (20) and the patient's face;
- outflow means (50) of gases expired by the patient for the exit from the volume of the gases expired by the patient; and
- an annular support frame (80);
- wherein the annular seal (30) is adapted to be compressed, in use, between the patient's face and the support frame (80);
- wherein the support frame (80) in use is removably associated with the shell (20) in proximity to the perimeter edge (23) thereof, and
- wherein the support frame (80) is configured to surround the patient's mouth, nose and eyes and comprises at least one fixing point (85) for the fixing of the support frame (80) to a neck strap (90).

2. The mask (10) according to claim 1 wherein the support frame (80) is configured to be fitted onto the shell (20) and exert a pressure on the perimeter edge (23) of the shell (20) against the patient's face.

3. The mask (10) according to claim 1, wherein the support frame (80) is rigid or semi-rigid.

4. The mask (10) according to claim 1, wherein the support frame (80) is made of a monolithic body.

5. The mask (10) according to claim 1, wherein the support frame (80) is made of various bodies joined together.

6. The mask (10) according to claim 1, wherein the support frame (80) has an inner face (81), facing towards the patient's face when worn by the patient, and an opposing outer face (82), wherein the inner face (81) is configured to define an abutment and/or a resting surface for at least one portion of the annular seal (30).

7. The mask (10) according to claim 6, wherein the inner face (81) is concave and defines a crib in which the annular seal (30) is at least partially removably housed,

8. The mask (10) according to claim 6 or 7, wherein the entire inner face (81) of the support frame (80) is configured to be brought into contact with the annular seal.

9. The mask (10) according to claim 1, wherein the annular seal (30) is inflatable.

10. The mask (10) according to claim 1, which comprises a neck strap (90) associated with the support frame (80) for the anchoring of the mask (10) to the patient's head.

## Patentansprüche

1. Vollgesichtsmaske (10) zur nicht-invasiven Atemtherapie für Patienten, die Folgendes umfasst:
- eine Schale (20), die mit einem geschlossenen Kreislaufumfangsrand (23) versehen ist, der eine ringförmige Dichtung (30) trägt, die dazu geeignet ist, in verstärkten und flüssigkeitsdichten Kontakt mit einem Bereich des Gesichts des Patienten zu kommen und Mund, Nase und Augen des Patienten umzugeben;
- Ansaugmittel (40) für ein atembares Gas, die an der Schale (20) angebracht sind, um atembares Gas in ein zwischen der Schale (20) und dem Gesicht des Patienten enthaltenes Volumen anzusaugen;
- Ausströmmittel (50) von durch den Patienten ausgeatmeten Gasen zum Austritt aus dem Volumen der durch den Patienten ausgeatmeten Gase; und
- einen ringförmigen Stützrahmen (80);
- wobei die ringförmige Dichtung (30) so angepasst ist, dass sie bei der Verwendung zwischen dem Gesicht des Patienten und dem Stützrahmen (80) zusammengedrückt wird;
- wobei der Stützrahmen (80) bei der Verwendung mit der Schale (20) in der Nähe des Umfangsrandes (23) derselben abnehmbar verbunden ist, und
- wobei der Stützrahmen (80) so ausgebildet ist, dass er den Mund, die Nase und die Augen des Patienten umgibt und mindestens einen Befestigungspunkt (85) zur Befestigung des Stützrahmens (80) an einem Nackenband (90) aufweist.

2. Maske (10) nach Anspruch 1, wobei der Stützrahmen (80) so ausgebildet ist, dass er auf die Schale (20) aufgesetzt wird und einen Druck auf den Umfangsrand (23) der Schale (20) gegen das Gesicht des Patienten ausübt.

3. Maske (10) nach Anspruch 1, wobei der Stützrahmen (80) starr oder halbstarr ist.

4. Maske (10) nach Anspruch 1, wobei der Stützrahmen (80) aus einem monolithischen Körper besteht.

5. Maske (10) nach Anspruch 1, wobei der Stützrahmen (80) aus verschiedenen, miteinander verbundenen Körpern besteht.

6. Maske (10) nach Anspruch 1
- wobei der Stützrahmen (80) eine innere Fläche (81), die dem Gesicht des Patienten zugewandt ist, wenn der Patient ihn anschaut, und eine gegenüberliegende äußere Fläche (82) aufweist, wobei die innere Fläche (81) so ausgebildet ist, dass sie ein Widerlager und/oder eine Auflagefläche für mindestens einen Teil der ringförmigen Dichtung (30) bestimmt.

7. Maske (10) nach Anspruch 6, wobei die innere Fläche (81) konkav ist und einen kleinen engen Raum bestimmt, in dem die ringförmige Dichtung (30) zumindest teilweise abnehmbar untergebracht ist,

8. Maske (10) nach Anspruch 6 oder 7,
wobei die gesamte innere Fläche (81) des Stützrahmens (80) so ausgebildet ist, dass sie mit der ringförmigen Dichtung in Kontakt gebracht wird.

9. Maske (10) nach Anspruch 1
- wobei die ringförmige Dichtung (30) aufblasbar ist.

10. Maske (10) nach Anspruch 1, die ein mit dem Stützrahmen (80) verbundenes Nackenband (90) zur Verankerung der Maske (10) am Kopf des Patienten umfasst.

## Revendications

1. Masque intégral (10) pour une thérapie respiratoire non invasive pour des patients, qui comprend :
- une coque (20) pourvue d'un bord périphérique en boucle fermée (23) supportant un joint annulaire (30) adapté pour venir en contact forcé et étanche aux fluides avec une zone du visage du patient et entourer la bouche, le nez et les yeux du patient ;
- des moyens d'admission (40) d'un gaz respirable placés sur la coque (20) pour l'admission du gaz respirable dans un volume compris entre la coque (20) et le visage du patient ;
- des moyens d'écoulement (50) des gaz expirés par le patient pour la sortie du volume des gaz expirés par le patient ; et
- un cadre de support annulaire (80) ;
- dans lequel le joint annulaire (30) est adapté pour être comprimé, lors de l'utilisation, entre le visage du patient et le cadre de support (80) ;
- dans lequel le cadre de support (80) lors de l'utilisation est associé de manière amovible à la coque (20) à proximité du bord périphérique (23) de celle-ci, et
- dans lequel le cadre de support (80) est configuré pour entourer la bouche, le nez et les yeux du patient et comprend au moins un point de fixation (85) pour la fixation du cadre de support (80) à une sangle de cou (90).

2. Masque (10) selon la revendication 1, dans lequel le cadre de support (80) est configuré pour être ajusté sur la coque (20) et exercer une pression sur le bord périphérique (23) de la coque (20) contre le visage du patient.

3. Masque (10) selon la revendication 1, dans lequel le cadre de support (80) est rigide ou semi-rigide.

4. Masque (10) selon la revendication 1, dans lequel le cadre de support (80) est constitué d'un corps monolithique.

5. Masque (10) selon la revendication 1, dans lequel le cadre de support (80) est constitué de divers corps reliés entre eux.

6. Masque (10) selon la revendication 1
- dans lequel le cadre de support (80) a une face interne (81), tournée vers le visage du patient lorsqu'il est porté par le patient, et une face externe opposée (82), dans lequel la face interne (81) est configurée pour définir une butée et/ou une surface d'appui pour au moins une partie du joint annulaire (30).

7. Masque (10) selon la revendication 6, dans lequel la face interne (81) est concave et définit un berceau dans lequel le joint annulaire (30) est au moins partiellement logé de manière amovible.

8. Masque (10) selon la revendication 6 ou 7,
dans lequel la totalité de la face intérieure (81) du cadre de support (80) est configurée pour être mis en contact avec le joint annulaire.

9. Masque (10) selon la revendication 1
- dans lequel le joint annulaire (30) est gonflable.

10. Masque (10) selon la revendication 1, **caractérisé en ce qu'**il comprend une sangle de cou (90) associée au cadre de support (80) pour l'ancrage du masque (10) sur la tête du patient.
